# FASCICULE DE BREVET EUROPEEN

(11) **EP 0 664 120 B1**
(45) Date de publication et mention de la délivrance du brevet: **26.04.2000**
(21) Numéro de dépôt: 94402974.3
(22) Date de dépôt: 21.12.1994
(51) Int. Cl.: A61K 31/135, A61K 9/00

(54) **Dispositif pour l'administration vaginale du tamoxifème et de ses analogues**
Vorrichtung für die vaginale Verabreichung von Tamoxifen und dessen Analogen
Device for the vaginal administration of tamoxifen and its analogs

(30) Priorité: 23.12.1993 GB 9326255
(43) Date de publication de la demande: 26.07.1995
(73) Titulaire: HOECHST MARION ROUSSEL, 92800 Puteaux (FR)
(72) Inventeur: Matlin, Stephen Alan, Birmingham, B29 7HU (GB); Wilkins, Andrew Thomas, Chippenham, Wiltshire SN15 4TQ (GB)
(74) Mandataire: Vieillefosse, Jean-Claude

(56) Documents cités:
- EP-A- 0 050 867
- WO-A-92/18101
- US-A- 4 235 988

## Description

Cette invention concerne des dispositifs médicaux spéciaux adaptés à la mise en place ou à l'implantation dans un organisme vivant. De tels dispositifs comprennent typiquement un composite, par ex. un anneau d'élastomère qui est inerte dans le milieu d'utilisation et un composé actif qui doit être libéré de la matrice inerte à une vitesse contrôlée pendant un temps prolongé. Ce type de préparation pharmaceutique est à différencier avec d'autres types de compositions pharmaceutiques qui libèrent le médicament par mise en solution dans le tractus gastro-intestinal comme dans un comprimé, une gélule ou une solution aqueuse.

Il est bien connu que les polymères silicone fournissent un support inerte à partir duquel un composé pharmaceutiquement actif peut être libéré dans un milieu environnant. Par exemple EP-A-050867 divulgue des préparations pharmaceutiques contenant un élastomère silicone et une substance pharmacologiquement active qui peuvent être implantées ou insérées dans une partie du corps où on désire que la substance pharmaceutiquement active soit libérée.

WO-92-18101 divulgue des anneaux vaginaux en matière silicone et une substance pharmacologiquement active qui sont utilisés dans le traitement de vaginose bactérienne.

Les dispositifs médicaux décrits dans l'art antérieur ne peuvent être appliqués qu'à des fins très spécifiques car ils présentent un problème en ce que la quantité de médicaments libérée à partir de la matrice de silicone est faible et des doses plus fortes ne peuvent pas être soutenues pendant un temps prolongé à un taux défini. En conséquence, il est nécessaire de remplacer fréquemment ces dispositifs par des préparations "fraîches" contenant une forte charge du médicament à administrer. Ceci se traduit par une dépense inutile et un manque de confort pour l'utilisatrice car ces préparations pharmaceutiques doivent être fréquemment mises en place et retirées de la partie du corps où le médicament doit être libérée.

Il y a récemment eu un grand intérêt dans le traitement du cancer, en particulier du cancer du sein.

Plusieurs méthodes de traitement ont été mises au point au cours des dernières années et des discussions ont également porté sur la prophylaxie du cancer du sein chez les personnes à haut risque.

Dans GB 1 064 629, un groupe de composés aromatiques a été divulgué : ce sont des substances fortement actives pour le traitement du cancer mais elles ont quelques effets secondaires importants lorsqu'ils sont administrés par voie orale.

L'invention suivante concerne des dispositifs médicaux spéciaux qui peuvent être avantageusement utilisés pour la prophylaxie et le traitement du cancer du sein d'origines diverses. De plus, les dispositifs médicaux peuvent être utilisés pour le traitement du carcinome pancréatique, du carcinome endométrial, du cancer de l'ovaire et du carcinome rénal.

Selon un aspect de la présente invention, on propose un dispositif médical destiné à être inséré dans une cavité vaginale comprenant un anneau d'élastomère contenant éventuellement un plastifiant et d'autres additifs, et contenant un composé pharmacologiquement actif de formule (I) : 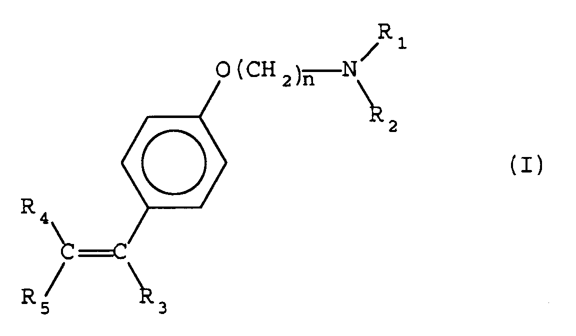 dans laquelle
R₁ représente l'hydrogène ou un radical alkyle contenant de 1 à 6 atomes de carbone ;
R₂ représente un radical alkyle contenant de 1 à 6 atomes de carbone ;
n représente un nombre entier de 2 à 6, de préférence 2 ;
R₃ et R₄, identiques ou différents, représentent chacun des radicaux aryle (C₆-C₁₂) qui peuvent porter éventuellement un ou deux substituants choisis parmi les atomes d'halogène, les radicaux alkyle (C₁-C₆) et alcoxy (C₁-C₆) ;
   et
R₅ représente un radical (C₁-C₆) :
   ou un sel pharmaceutiquement acceptable de celui-ci.

Comme composé pharmacologiquement actif, on utilise de préférence une substance de formule (Ia) connue sous le nom de tamoxifène :

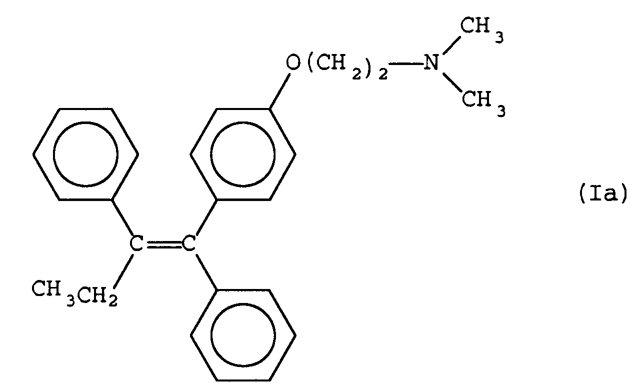

On peut également utiliser un sel pharmaceutiquement acceptable de tamoxifène, par ex. le citrate de tamoxifène.

L'utilisation de certains sels lipophiles peut améliorer à la fois la libération à partir du dispositif et l'absorption dans la circulation générale. Des exemples de tels sels incluent les sels de tamoxifène avec l'acide pamoïque (C₂₃H₁₆O₆) et l'acide stéarique (C₁₈H₃₆O₂).

L'anneau d'élastomère selon l'invention est de préférence constitué d'un élastomère silicone ; toutefois, on peut utiliser d'autes élastomères tels que polyéthylène acétate de cellulose ou des copolymères à base d'éthylène et d'acétate de vinyle.

L'élastomère silicone utilisé dans la présente invention peut être tout élastomère de qualité médicale qui est adapté à une utilisation comme matrice pour médicament destinée à l'encapsulation d'un médicament à administrer par libération contrôlée et qui est inerte dans le milieu d'utilisation.

Des anneaux vaginaux peuvent également être réalisés avec une combinaison d'élastomères silicone et thermoplastiques, par exemple un dispositif peut avoir un noyau fabriqué à partir d'une suspension de médicament dans de l'éthylène acétate de vinyle et une enveloppe formée de silicone.

Les élastomères silicone particulièrement préférables sont ceux de qualité médicale constitués de chaînes polydiméthylsiloxane contenant des fonctions silanol (-OH). Un agent de réticulation à ester-silicate, par ex. le n-propyl-orthosilicate, est incorporé dans la formulation d'élastomère à un taux compris entre 0,1 et 20% de préférence entre 1 et 5% en poids. L'élastomère préféré contient soit une charge de terre de diatomées soit une charge de silice fondue à des taux compris entre 5 et 60%, de préférence 20 et 26% en poids. La réticulation de l'élastomère est facilitée par l'utilisation de catalyseur à l'étain(II) tel que l'acide 2-éthylhexanoïque d'étain(II) qui est ajouté à l'élastomère dans des rapports compris entre 1 et 16 parties en poids de catalyseur à 800 parties en poids d'élastomère. De tels élastomères du commerce actuellement disponibles sont par ex. le SILASTIC® QCF 7 3099 (désigné également X7-3099 et Q7-3099) et le SILASTIC® X7-4977 (Dow Corning Corporation, Valbonne, France).

De manière alternative, on peut utiliser une qualité de silicone qui inclut des fonctions vinyle (Si-CH=CH₂) dans la structure de polydiméthylsiloxane qui deviennent réticulées après traitement par un catalyseur au platine. Des exemples de tels polymères sont le SILASTIC® MDX4-4210, le SILASTIC® Q7-4735 et le SILASTIC® Q7-4750.

En outre, on peut utiliser des silicones où les chaînes de polydiméthylsiloxane sont réticulées après traitement avec un radical libre, par ex. un radical peroxyde.

Les ingrédients pharmacologiquement actifs qui peuvent être utilisés dans la présente invention sont des composés de formule (I), mais de préférence le composé de formule (Ia). Ces composés dont facilement disponibles et peuvent être préparés comme divulgué dans GB 1 064 629.

La quantité de composé pharmacologiquement actif chargé dans l'élastomère silicone n'est pas limitée et peut être contenue dans la préparation pharmaceutique de la présente invention dans des quantités de 30% en poids ou plus de dispositif médical, le taux de charge de l'ingrédient pharmacologiquement actif étant basé uniquement sur la compatibilité de l'ingrédient de formule (I) avec la matrice d'élastomère silicone. De préférence, le composé actif est contenu dans le dispositif médical selon l'invention dans une quantité de 1 à 30 %, de préférence notamment de 10 à 25% en poids sur la base de poids du dispositif médical.

Des additifs peuvent être ajoutés au polymère pour aider à contrôler la libération du médicament. Les additifs utilisés dans la présente invention doivent être compatibles avec l'élastomère et compatibles avec le composé pharmacologiquement actif de formule (I) devant être contenu dans l'élastomère. Ceux-ci incluent :
- des agents favorisant la pénétration, par ex. l'huile de coco, l'Azone et l'huile végétale,
- des inhibiteurs de pénétration, par ex. la méthylcellulose et le lactose,
- des agents osmotiques (pour absorber de l'eau dans le dispositif), par ex. des sels tels que le chlorure de sodium, le chlorure de potassium et le phosphate de sodium,
- des agents de réglage du pH, par ex. l'hydrogénoorthophosphate disodique, Na₂HPO₄,
- des agents modificateurs de viscosité, par ex. un fluide silicone de faible poids moléculaire tel que le fluide médical 360 de Dow Corning.

Les additifs ci-dessus doivent être incorporés dans le dispositif à des taux compris entre 0 et 50%, de préférence de 2 à 20% en poids.

Le dispositif médical comprenant l'anneau d'élastomère a une dimension externe moyenne de 2 à 10 cm ; la taille exacte est fonction de l'application particulière. Les anneaux vaginaux ont normalement un diamètre externe de 4 à 8 cm, de préférence de 5 à 7 cm de manière tout à fait préférée de 5 à 6 cm.

Le dispositif médical a normalement un poids total minimum de 0,9 g et un poids total maximum de 110 g. Les anneaux vaginaux préférés pèsent de 5 à 16 g, et ils peuvent être utilisés comme source de composé actif pendant plusieurs mois.

La largeur de la section transversale de l'anneau est normalement de 0,4 à 2,0 cm, de préférence de 0,6 à 1,0 cm. Le dispositif médical selon cette invention peut être réalisé en différents types de géométrie. En pratique, on préfère notamment, pour une application intra-vaginale, l'anneau de type noyau et les dispositifs de type coque.

Dans un anneaux vaginal de type noyau, le noyau est produit à partir d'une suspension du composé actif dans l'élastomère, ceci forme le réservoir de médicament. Le noyau est enrobé de une ou plusieurs couches de matière polymère ne contenant de préférence aucun composé actif. Cet enrobage aide à contrôler la vitesse de délivrance de médicament. Le noyau peut passer au travers de l'anneau en continu ou il peut présenter une longueur partielle ou être constitué de plusieurs parties.

Le dispositif médical est de préférence un anneau vaginal multicouche contenant un anneau de support dépourvu du composé actif, une deuxième couche, qui est le réservoir de médicament et contient le composé actif de formule (I), et à titre de troisième couche, une couche enrobante qui recouvre la couche de substance active. Ce type de dispositif, appelé l'anneau de type coque, est utilisé lorsqu'on préfère des noyaux de grand diamètre pour maintenir une libération suffisante du composé actif, et lorsqu'on doit éviter un gaspillage inutile de composé actif.

En principe, il est également possible d'utiliser un anneau vaginal de type matrice qui est réalisé à partir d'une suspension homogène de composé actif dans un polymère. L'anneau de type matrice libère la plus forte dose de composé actif. Un autre type d'anneau vaginal est constitué d'un noyau contral contenant l'élastomère sans aucun médicament et un enrobage contenant le composé actif et l'élastomère (suspension). Le noyau central peut également ne contenir que de l'air.

Différentes méthodes peuvent être utilisées pour la préparation des dispositifs médicaux.

Selon un autres aspect de la présente invention, on propose un procédé de préparation d'un dispositif médical comprenant un anneau d'élastomère qui contient un composé pharmacologiquement actif de formule (I) comme décrit ci-dessus en utilisant un procédé de moulage par injection ou d'extrusion.

La technique de moulage par injection ou d'extrusion par exemple peut être utilisée pour préparer les anneaux d'élastomère. Pour le procédé de moulage par injection, le polymère fluide est injecté dans un moule de forme souhaitée, par ex. un moule toroïdal. Le polymère fluide se durcit dans le moule soit par réaction de réticulation soit par refroidissement de la masse fondue (par ex. pour l'éthylène acétate de vinyle). Le procédé général peut être modifié pour produire les autres types de dispositifs décrits ci-dessus.

Le procédé d'extrusion implique le fait de pousser une matière polymère visqueuse à travers une filière pour produire une matière de longueur continue appelée un extrudat. La co-extrusion implique l'extrusion simultanée de deux ou plusieurs matières différentes qui s'associent dans un manifold pour produire une structure multicouche. Pour la préparation d'anneaux vaginaux, l'extrudat cylindrique doit être découpé en longueurs et les deux bouts reliés. Ceci peut être réalisé en plaçant dans des moules toroïdaux qui sont chauffés pour effectuer la réticulation.

Les dispositifs médicaux selon cette invention peuvent également être réalisés par une technique de pultrusion dans laquelle un noyau central est extrudé séparément et cette longueur unique de matière est renvoyée dans une extrudeuse où une couche externe est extrudée autour d'elle. De manière alternative, une enveloppe de polymère pourrait être injectée autour d'un noyau extrudé.

Selon un autre aspect de la présente invention, on propose une méthode de traitement ou la thérapeutique prophylactique du cancer chez un sujet comprenant l'insertion d'un dispositif médical comme décrit précédemment dans le vagin dudit sujet. La méthode de traitement ou de thérapeutique prophylactique préférée est celle du cancer du sein.

L'invention est encore illustrée par les exemples suivants.

### EXEMPLE 1

### Préparation d'un anneau vaginal contenant du tamoxifène.

512,89 g du polymère de base Silastic® 382 (moins le catalyseur) ont été préparés en mélangeant 500 g de Silastic® QCF 7 3099 avec 12,89 g de propylorthosilicate à l'aide d'un agitateur à pales. Le mélange a été dégazé sous vide.

Le mélange médicament/polymère a été préparé en ajoutant 1,0 g de tamoxifène en poudre directement à 3 g de base de polymère (moins le catalyseur) et en mélangeant avec un agitateur à pales. Le mélange a ensuite été dégazé sous vide.

La fabrication des anneaux a été réalisée par moulage par injection. La base de polymère et le mélange médicament polymère ont été activés avec le catalyseur à l'octoate stanneux immédiatement avant chaque injection. Les moules ont été réalisés à partir de polyméthylméthacrylate et les produits moulés ont été cuits à 600°C pendant 15 minutes.

Deux dispositifs ont été produits. L'un était un anneau de type noyau qui contenait un noyau de 4,4 mm de diamètre. L'autre était un anneau de type coque qui contenait un réservoir de médicament de 8,3 mm de diamètre. L'anneau conçu selon le type coque contenait un noyau interne inerte de 5,4 mm. Les deux dispositifs avaient des dimensions externes de 55 mm de diamètre global et de 9,5 mm de diamètre transversal. La cuisson aurait pu être effectuée à une température plus élevée pendant un temps plus court si des moules métalliques avaient été utilisés, par ex. 2 minutes à 80°C.

Le dispositif à noyau de 4,4 mm de diamètre contenait 0,58 g de composé actif. Le dispositif à coque de 8,3 mm de diamètre contenait 1,25 g de composé actif. Le poids total de chaque anneau était de 11 g.

La voie vaginale pour la délivrance du tamoxifène présente plusieurs avantages par rapport à l'administration par voie orale :
a) La libération continue évite des pics de concentration qui se produisent suivant l'administration par voie orale du composé actif.
b) Une meilleure observance thérapeutique. Ceci est particulièrement important lorsque le composé est utilisé en thérapeutique prophylactique où il existe très peu de motivation pour une patiente "saine" de se rappeler de prendre des comprimés tous les jours.
c) Un effet secondaire courant de l'absorption par voie orale du comprimé contenant un composé de formule (I) est un dérangement gastro-intestinal. Un anneau vaginal aide les patientes souffrant de problèmes de tolérance avec les comprimés.
d) Le tamoxifène délivré par le vagin conduit à une bio-disponibilité accrue par rapport à la voie orale, ce qui conduit à un plus faible risque d'effets secondaires.

### EXEMPLE 2

Le taux de libération de tamoxifène à partir du dispositif à noyau de 4,4 mm de diamètre a été déterminé in vitro par suspension de l'anneau dans 500 ml de milieu d'élution contenu dans un flacon de verre fermé. Le flacon a été maintenu à la température du corps (37°C) dans un bain-marie et l'agitation a été assurée par un barreau magnétique revêtu de PTFE qui était actionné par un bloc agitateur submersible.

Le milieu d'élution a été préparé en ajustant une solution de dihydrogéno-orthophosphate de potassium 0,1 M (KH₂PO₄, qualité AnalaR, BDH, Poole, GB) à pH 3 avec de l'acide orthophosphorique.

Des prélèvements du milieu d'élution ont été réalisés toutes les 24 heures et analysés pour déterminer la quantité de tamoxifène libéré.

Immédiatement après l'échantillonnage, le milieu d'élution a été remplacé par du milieu frais. Ceci a évité une accumulation du médicament dans le milieu qui autrement aurait pu ralentir le taux de libération de médicament à partir du dispositif. Ceci mime la situation in vivo où un flux sanguin constant assure l'élimination efficace du médicament de l'environnement local du dispositif.

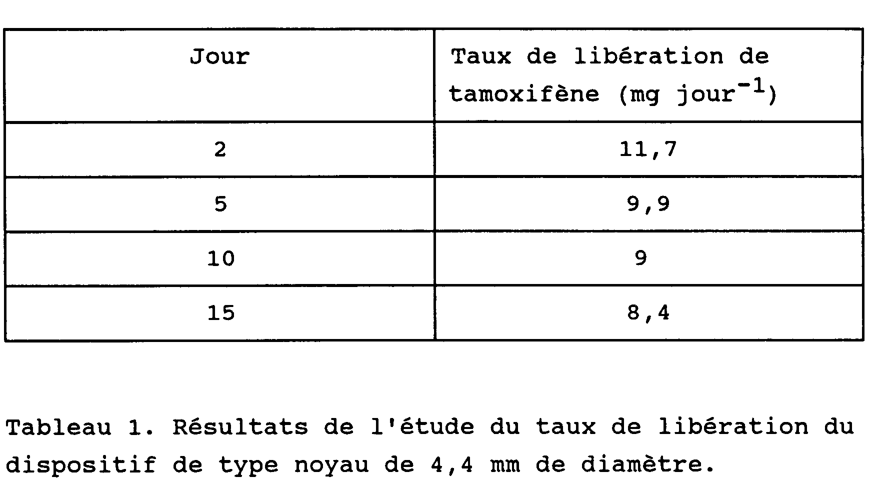

### EXEMPLE 3

En raison du fait que le dispositif de type coque de 8,3 mm de diamètre a été conçu pour une forte libération de médicament, un remplacement fréquent du milieu d'élution était nécessaire. Ceci a été réalisé à l'aide d'un système de circulation. L'anneau a été mis en suspension dans du milieu d'élution dans un flacon comme décrit à l'exemple 1. En outre, deux tubes ont été scellés dans le bouchon. Un tube a servi d'entrée pour admettre du milieu dans le flacon et l'autre de sortie pour permettre au milieu de sortir du flacon et de couler vers un récipient de collection d'effluent. L'écoulement du milieu a été induit par une pompe péristaltique.

Toutes les 24 heures, le milieu dans le flacon a été vidé dans le récipient de collection d'effluent et celui-ci a été remplacé par du milieu frais. Le volume de l'effluent a été déterminé et l'effluent prélevé et analysé pour déterminer la quantité de tamoxifène libéré. L'écoulement de milieu était compris entre 2 et 3 litres par 24 heures.

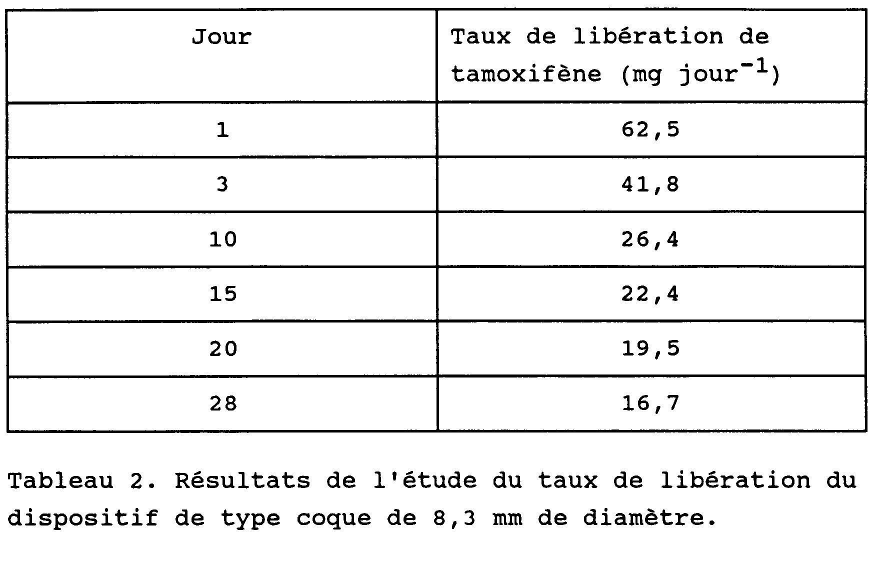

Les méthodes d'essai de libération in vitro utilisées aux exemples 2 et 3 se sont révélées donner une bonne corrélation in vivo lorsqu'elles ont été utilisées pour tester des anneaux vaginaux contraceptifs libérant de la progestérone (voir S.A. Matlin, A. Belenguer, P.E. Hall. Progesterone-releasing vaginal rings for use in postpartum contraception. I. In vitro release rates progesterone from core-loaded rings, (Anneaux vaginaux libérant de la progestérone destinée à être utilisée en contraception post-partum. I. Taux de libération in vitro de progestérone à partir d'anneaux chargés au noyau). Contraception, 1992, 45, 329,341 ; et B.M. Landgren, P.E. Hall, S.Z. Cekan. Progesterone-releasing vaginal rings for use in postpartum contraception. II. Pharmacokinetic profiles in women. (Anneaux vaginaux libérant de la progestérone destinée à être utilisée en contraception post-partum. II. Profils pharmacocinétiques chez la femme.) Contraception, 1992, 45, 343-349).

### EXEMPLE 4

Des anneaux vaginaux correspondants peuvent être préparés en utilisant des sels de tamoxifène tels que le citrate ou d'autres composés de formule générale (I).

## Revendications

1. Dispositif médical destiné à être inséré dans une cavité vaginale comprenant un anneau d'élastomère silicone contenant un composé pharmacologiquement actif de formule (I) : 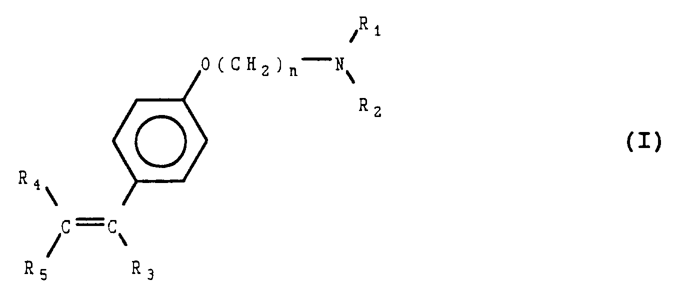 dans laquelle
R₁ représente l'hydrogène ou un radical alkyle contenant de 1 à 6 atomes de carbone ;
R₂ représente un radical alkyle contenant de 1 à 6 atomes de carbone ;
n représente un nombre entier de 2 à 6, de préférence 2 ;
R₃ et R₄, identiques ou différents, représentent chacun des radicaux aryle (C₆-C₁₂) qui peuvent porter éventuellement un ou deux substituants choisis parmi les atomes d'halogène, les radicaux alkyle (C₁-C₆) et alcoxy (C₁-C₆) ;
et
R₅ représente un radical (C₁-C₆) :
ou un sel pharmaceutiquement acceptable de celui-ci.

2. Dispositif médical selon la revendication 1, caractérisé en ce que le composé pharmacologiquement actif est un composé de formule (Ia) : 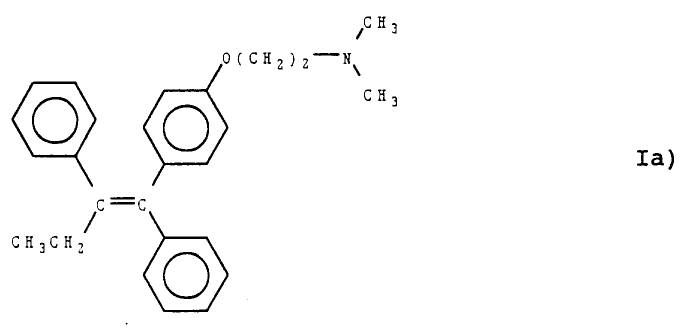 ou un sel pharmaceutiquement acceptable de celui-ci.

3. Dispositif médical selon la revendication 1 ou 2, caractérisé en ce que l'anneau d'élastomère est constitué d'un élastomère silicone qui contient le composé actif dans une quantité de 1 à 30% en poids par rapport au poids du dispositif médical.

4. Dispositif médical selon la revendication 1, 2 ou 3, caractérisé en ce que le composé actif est contenu dans l'anneau d'élastomère silicone dans une quantité de 10 à 25% en poids par rapport au poids du dispositif médical.

5. Dispositif médical selon les revendications 1 à 4, caractérisé en ce que l'anneau d'élastomère est un anneau de type coque ou de type noyau d'un élastomère silicone, contenant de 10 à 25% en poids du composé pharmacologiquement actif par rapport au poids du dispositif médical.

6. Procédé de préparation d'un dispositif médical comprenant un anneau d'élastomère silicone qui contient un composé pharmacologiquement actif de formule (I) comme décrit dans la revendication 1 en utilisant un procédé de moulage par injection ou d'extrusion.

## Claims

1. Medical device intended to be inserted into a vaginal cavity comprising a silicone elastomer ring containing a pharmacologically active compound of formula (I): 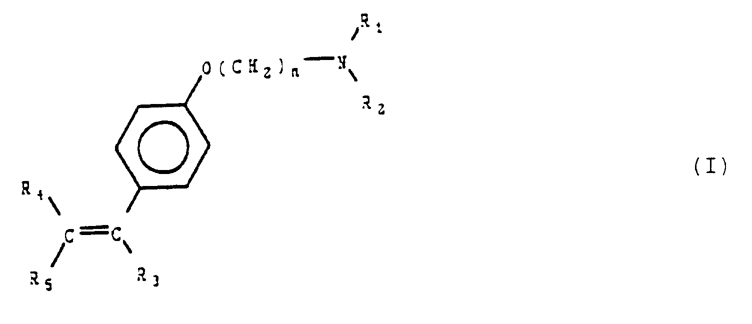 in which
R₁ represents hydrogen or an alkyl radical containing 1 to 6 carbon atoms;
R₂ represents an alkyl radical containing 1 to 6 carbon atoms;
n represents an integer from 2 to 6, preferably 2;
R₃ and R₄, identical or different, each represent (C₆-C₁₂) aryl radicals which can optionally carry one or two substituents chosen from halogen atoms, the (C₁-C₆) alkyl and (C₁-C₆) alkoxy radicals;
and
R₅ represents a (C₁-C₆) radical:
or a pharmaceutically acceptable salt of the latter.

2. Medical device according to claim 1, characterized in that the pharmacologically active compound is a compound of formula (Ia): 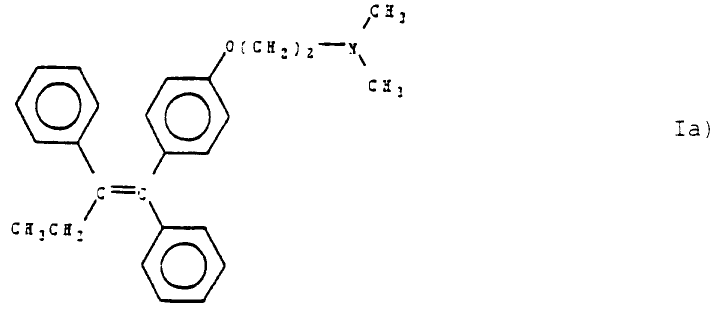 or a pharmaceutically acceptable salt of the latter.

3. Medical device according to claim 1 or 2, characterized in that the elastomer rang is constituted by a silicone elastomer which contains the active compound in a quantity of 1 to 30% by weight with respect to the weight of the medical device.

4. Medical device according to claim 1, 2 or 3, characterized in that the active compound is contained in the silicone elastomer ring in a quantity of 10 to 25% by weight with respect to the weight of the medical device.

5. Medical device according to claims 1 to 4, characterized in that the elastomer ring is a shell-type or core-type ring of a silicone elastomer, containing 10 to 25% by weight of pharmacologically active compound with respect to the weight of the medical device.

6. Process for the preparation of a medical device comprising a silicone elastomer ring which contains a pharmacologically active compound of formula (I) as described in claim 1 using a moulding process by injection or extrusion.

## Patentansprüche

1. Medizinische Vorrichtung zur Insertion in eine Vaginalhöhle, umfassend einen Siliconelastomerring, der eine pharmakologisch aktive Verbindung der Formel (I): 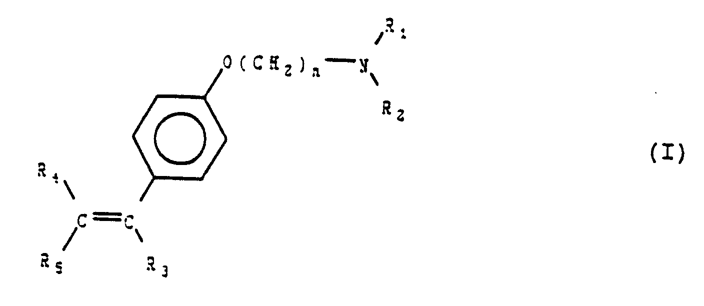
worin R₁ Wasserstoff oder einen Alkylrest mit 1 bis 6 Kohlenstoffatomen bezeichnet,
R₂ einen Alkylrest mit 1 bis 6 Kohlenstoffatomen bezeichnet,
n eine ganze Zahl von 2 bis 6, bevorzugt 2, bezeichnet,
R₃ und R₄, die gleich oder verschieden sind, jeweils einen C₆-C₁₂-Arylrest bezeichnen, der gegebenenfalls einen oder zwei Substituenten, ausgewählt aus Halogenatomen, C₁-C₆-Alkylresten und C₁-C₆-Alkoxyresten, tragen kann, und
R₅ einen C₁-C₆-Rest bezeichnet,
oder ein pharmazeutisch verträgliches Salz davon enthält.

2. Medizinische Vorrichtung nach Anspruch 1, dadurch gekennzeichnet, daß die pharmakologisch aktive Verbindung eine Verbindung der Formel (Ia) 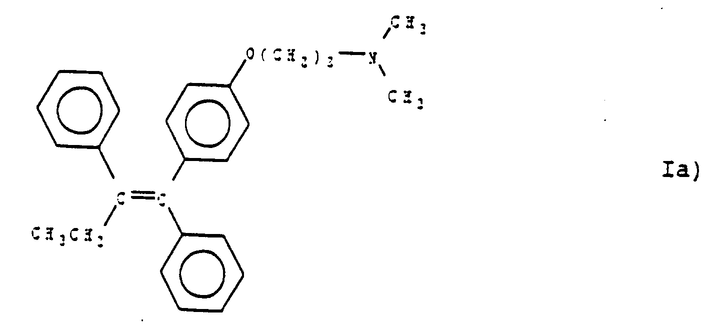 oder ein pharmazeutisch verträgliches Salz davon ist.

3. Medizinische Vorrichtung nach Anspruch 1 oder 2, dadurch gekennzeichnet, daß der Elastomerring aus einem Siliconelastomeren besteht, das die aktive Verbindung in einer Menge von 1 bis 30 Gew.-% bezogen auf das Gewicht der medizinischen Vorrichtung enthält.

4. Medizinische Vorrichtung nach Anspruch 1, 2 oder 3, dadurch gekennzeichnet, daß die aktive Verbindung in dem Siliconelastomerring in einer Menge von 10 bis 25 Gew.-% bezogen auf das Gewicht der medizinischen Vorrichtung enthalten ist.

5. Medizinische Vorrichtung nach den Ansprüchen 1 bis 4, dadurch gekennzeichnet, daß der Elastomerring ein Ring vom Typ Schale oder vom Typ Kern eines Siliconelastomeren ist, das 10 bis 25 Gew.-% der pharmakologisch aktiven Verbindung bezogen auf das Gewicht der medizinischen Vorrichtung enthält.

6. Verfahren zur Herstellung einer medizinischen Vorrichtung die einen Siliconelastomerring umfasst, der eine pharmakologisch aktive Verbindung der Formel (I) wie in Anspruch 1 beschrieben enthält, wobei man ein Formgebungsverfahren durch Injektion oder durch Extrusion verwendet.
